(19) Europäisches Patentamt
European Patent Office
Office européen des brevets

(11) Veröffentlichungsnummer: **0 304 685 B1**

# (12) EUROPÄISCHE PATENTSCHRIFT

(45) Veröffentlichungstag der Patentschrift: **16.10.91**

(51) Int. Cl.5: **C07D 223/10**, C07D 201/08

(21) Anmeldenummer: **88112587.6**

(22) Anmeldetag: **03.08.88**

## (54) Verfahren zur Herstellung von N-alkylierten Caprolactamen.

(30) Priorität: **15.08.87 DE 3727255**
**23.10.87 DE 3735904**

(43) Veröffentlichungstag der Anmeldung:
**01.03.89 Patentblatt 89/09**

(45) Bekanntmachung des Hinweises auf die Patenterteilung:
**16.10.91 Patentblatt 91/42**

(84) Benannte Vertragsstaaten:
**BE DE FR GB IT**

(56) Entgegenhaltungen:
**DE-A- 1 944 910**
**DE-A- 2 131 199**

**JOURNAL OF THE AMERICAN CHEMICAL SOCIETY Band 94, Nr. 23, 15. November 1972, Seiten 8142-8147; K.L. KIRK et al.: "Intramolecular aminolysis of amides. Effects of electronic variation in the attacking and leaving groups"**

**T.H. Lowry/K.S. Richardson, MECHANISMEN UND THEORIE IN DER ORGANISCHEN CHEMIE, Verlag Chemie, Weinheim, 1980, Seiten 452-460**

**JOURNAL OF THE AMERICAN CHEMICAL SOCIETY Band 70, Nr. 6, Juni 1948, Seite 2118; R.E. BENSON et al.**

(73) Patentinhaber: **BAYER AG**

**W-5090 Leverkusen 1 Bayerwerk(DE)**

(72) Erfinder: **Jakob, Wolfgang**
**Am Domacker 81**
**W-4130 Moers(DE)**
Erfinder: **Alewelt, Wolfgang, Dr.**
**Stratumer Feld 17**
**W-4150 Krefeld 12(DE)**
Erfinder: **Tresper, Erhard, Dr.**
**Moerser Strasse 394**
**W-4150 Krefeld 11(DE)**

EP 0 304 685 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft ein Verfahren zur Herstellung von N-alkylierten Caprolactamen durch Cyclisierung von N-alkylierten Capronsäuren.

N-alkylierte Caprolactame gehören zu der Gruppe der aprotisch dipolaren Lösungsmittel und werden häufig bei der Durchführung chemischer Synthesen vor allem im großtechnischen Maßstab eingesetzt. Sie werden z.B. bei der Herstellung von Polyarylensulfiden bevorzugt verwendet (z.B. EP-OS 17 10 21).

Die N-alkylierten Caprolactame haben im Vergleich zu anderen Lösungsmitteln wie Pyrrolidonen den Vorteil höherer Siedepunkte. Daher kann bei Verwendung von N-alkylierten Caprolactamen als Lösungsmittel eine gegebenenfalls notwendige hohe Reaktionstemperatur unter Normaldruck erreicht werden, was die Wirtschaftlichkeit des Verfahrens, z.B. im technischen Maßstab erhöht.

In Gegenwart von Alkali werden jedoch auch die N-alkylierten Caprolactame teilweise gespalten. Es entstehen als unerwünschte Nebenprodukte N-Alkylaminocapronate.

Aus Am. Soc. 70 (1948) 2118, ist die partielle Cyclisierung von N-Alkylaminocapronsäuren zu N-Alkylcaprolactamen durch Erhitzen der Säuren über ihren Schmelzpunkt und gleichzeitiges Abdestillieren von Lactam und Wasser bekannt. Jedoch sind die Ausbeuten mit ca. 80 % für einen technischen Maßstab zu gering.

Es wurde nun gefunden, daß man zu über 95 % N-Alkylcaprolactame durch Cyclisierung von N-alkylierten Capronsäuren und N-Alkylaminocapronaten erhalten kann, wenn deren Lösungen auf den isoelektrischen Punkt der jeweiligen N-Alkylaminocapronsäure eingestellt, auf ca. 230° C erhitzt und gegebenenfalls destilliert werden.

N-alkylierte Lactame aus N-Alkylaminocapronsäuren oder deren Salzen nahezu quantitativ durch Cyclisierung herzustellen, war nicht zu erwarten, da die Bildung von 7-gliedrigen Ringen durch Cyclisierung nur erschwert und nicht mit befriedigenden Ausbeuten zu erreichen ist.

Insbesondere erlaubt das erfindungsgemäße Verfahren aus den bei chemischen Umsetzungen anfallenden Mutterlaugen, die die N-Alkylaminocapronate enthalten und die freien Säuren enthalten, N-Alkylcaprolactame zurückzugewinnen.

Gegenstand der Erfindung ist daher ein Verfahren zur Herstellung von N-Alkylcaprolactamen durch Cyclisierung von N-Alkylaminocapronsäuren oder deren Salze der Formel (I)

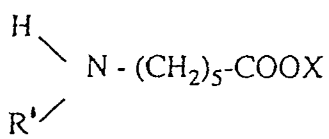

(I),

in welcher

$R^1$ für $C_1$-$C_{20}$-Alkyl und $C_5$-$C_{14}$-Cycloalkyl und X für H oder ein Kation wie Amnionium, Li, Na, K, 1/2 Ca, 1/2 Mg steht,
dadurch gekennzeichnet, daß man Lösungen von 0,1-90 Gew.-% von N-Alkylaminocapronsäuren oder deren Salzen in den korrespondierenden N-Alkylaminocaprolactamen auf den isoelektrischen Punkt der jeweiligen N-Alkylaminocaprosäure einstellt, die Lösungen kurzzeitig auf den jeweiligen Siedepunkt des N-Alkylaminocaprolactams erhitzt in einem Temperaturbereich von 180 - 300° C und nach Abdestillieren des entstandenen Reaktionswassers gegebenenfalls das N-Aminocaprolactam wieder unmittelbar in eine Umsetzungslösung zurückführt oder das Lactam durch Destillation abtrennt und reinigt.

Bevorzugt wird N-Methylaminocapronsäure bzw. deren Na-Salz.

Bei der Durchführung des erfindungsgemäßen Verfahrens wird nach Einstellen der Reaktionslösung auf den isoelektrischen Punkt der jeweiligen Aminosäure auf eine Temperatur von 180-300° C, bevorzugt 200-240° C, insbesondere bei 230° C aufgeheizt. Der Druck beträgt 0,01-40 bar. Bevorzugt wird ein Druck, der sich durch den Dampfdruck der Lösung einstellt, besonders bevorzugt ist Normaldruck.

Die Konzentration der N-Alkylaminocapronsäuren oder deren Salze in den korrepondierenden N-Alkylcaprolactamen kann 0,1-90 Gew.-%, bevorzugt 5-20 Gew.-%, insbesondere 10-15 Gew.-% betragen.

## Beispiele

Vergleichsbeispiel 1

50 g N-Methylaminocapronsäure werden in einem 100 ml Dreihalskolben mit Innenthermometer und absteigendem Kühler auf 250° C erhitzt. Dabei destillieren 5,8 g Wasser ab. Danach wird abgekühlt und bei ca. 0,3 bar das gebildete N-Methylcaprolactam abdestilliert. Man erhält 33,7 g Destillat (77 % Ausbeute), das gaschromatographisch und IR-spektroskopisch als N-Methylcaprolactam identifiziert wird und 9,7 g undestillierbaren Rückstand.

Vergleichsbeispiel 2

50 g Natrium-N-Methylaminocapronat wurden wie in Beispiel 1 beschrieben behandelt. Es war kein Destillat faßbar und der Rückstand betrug 49,9 g,

Beispiel 1

50 g N-Methylaminocapronsäure werden in 100 g N-Methylcaprolactam gelöst und in einem 250 ml Dreihalskolben mit Innenthermometer und absteigendem Kühler 3 Stunden auf 230° C erhitzt. Dabei destillieren 3,0 g Wasser ab. Danach wird abgekühlt und bei ca. 0,3 bar bis 245° C Sumpftemperatur destilliert. Es werden 121 g (96 % Ausbeute) Destillat erhalten, der Rückstand betrug 0,6 g.

Beispiel 2

In einem 250 ml Dreihalskolben mit Innenthermometer und absteigendem Kühler werden 30 g Natrium-N-methylaminocapronat in 100 g N-Methylcaprolactam suspendiert. Die Suspension wird mit 36 ml 5 n Salzsäure versetzt. Die entstandene Lösung hat einen pH-Wert von 6,7. Nach Destillation wie in Beispiel 1 erhält man 122 g (96,5 %) N-Methylcaprolactam.

**Patentansprüche**

1. Verfahren zur Herstellung Von N-Alkylcaprolactamen durch Cyclisierung von N-Alkylaminocapronsäuren oder deren Salze der Formel (I)

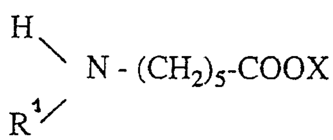

(I),

in welcher

$R^1$ für $C_1$-$C_{20}$-Alkyl und $C_5$-$C_{14}$-Cycloalkyl und X für H oder ein Kation wie Ammonium, Li, Na, K, 1/2 Ca, 1/2 Mg steht,

dadurch gekennzeichnet, daß man Lösungen von 0,1-90 Gew.-% von N-Alkylaminocapronsäuren oder deren Salzen in den kortespondierenden N-Alkylaminocaprolactamen auf den isoelektrischen Punkt der jeweiligen N-Alkylaminocaprosäure einstellt, die Lösungen kurzzeitig auf den jeweiligen Siedepunkt des N-Alkylaminocaprolactams erhitzt in einem Temperaturbereich von 180 - 300°C und nach Abdestillieren des entstandenen Reaktionswassers gegebenenfalls das N-Aminocaprolactam wieder unmittelbar in eine Umsetzungslösung zurückführt oder das Lactam durch Destillation abtrennt und reinigt.

2. Verfahren nach Anspruch 1, dadurch gekenzeicnnet, daß bei Drucken von 0,01 - 40 bar cyclisiert wird.

**Claims**

1. A process for the production of N-alkyl caprolactams by cyclization of N-alkylaminocaproic acids or salts thereof corresponding to formula (I)

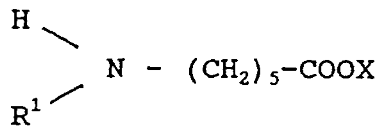

(I),

in which

$R^1$ represents $C_{1-20}$ alkyl and $C_{1-20}$ cycloalkyl and

X represents H or a cation, such as ammonium, Li, Na, K, 1/2 Ca, 1/2 Mg,

characterized in that solutions of 0.1 - 90% by weight of N-alkylaminocaproic acids or salts thereof in the corresponding N-alkylaminocaprolactams are adjusted to the isoelectric point of the particular N-alkylaminocaproic acid, the solutions are briefly heated to the particular boiling point of the N-alkylaminocaprolactam in the temperature range from 180 to 300 °C and, after the water of reaction formed has been distilled off, the N-aminocaprolactam is optionally reintroduced immediately into a reaction solution or the lactam is separated off by distillation and purified.

**2.** A process as claimed in claim 1, characterized in that the cyclization is carried out under pressures of 0.01 to 40 bar.

## Revendications

**1.** Procédé de production de N-alkylcaprolactames par cyclisation d'acides N-alkylaminocaproïques ou de leurs sels de formule (I)

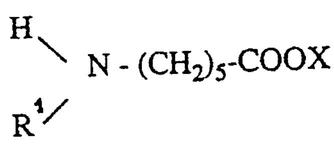

(I)

dans laquelle

$R^1$ représente un groupe alkyle en $C_1$ à $C_{20}$ et un groupe cycloalkyle en $C_5$ à $C_{14}$, et X est l'hydrogène ou un cation tel gu'ammonium, Li, Na, K, 1/2 Ca, 1/2 Mg, caractérisé en ce qu'on ajuste des solutions de 0,1-90 % en poids d'acides N-alkylaminocaproïques ou de leurs sels dans les N-alkylaminocaprolactames correspondants au point isoélectrique de l'acide N-alkylaminocaproïque concerné, on chauffe brièvement les solutions au point d'ébullition correspondant du N-alkylaminocaprolactame dans une plage de températures de 180 à 300 °C et, après élimination par distillation de l'eau de réaction formée, on recycle éventuellement le N-aminocaprolactame directement dans une solution réactionnelle ou bien on sépare le lactame par distillation et on le purifie.

**2.** Procédé suivant la revendication 1, caractérisé en ce qu'on conduit la cyclisation à des pressions de 0,01 à 40 bars.